# FASCICULE DE BREVET EUROPEEN

(11) **EP 0 897 310 B1**
(45) Date de publication et mention de la délivrance du brevet: **05.02.2003**
(21) Numéro de dépôt: 97921912.8
(22) Date de dépôt: 30.04.1997
(51) Int. Cl.: A61M 39/28, F16K 7/06

(54) **DISPOSITF D'OBTURATION PAR PINCEMENT D'UN TUBE SOUPLE**
QUETSCHVENTIL ZUM ABSPERREN EINES SCHLAUCHS
PINCH OBTURATING DEVICE FOR FLEXIBLE TUBE

(30) Priorité: 03.05.1996 FR 9605543
(43) Date de publication de la demande: 24.02.1999
(73) Titulaire: DEBIOTECH S.A., 1000 Lausanne 9 (CH)
(72) Inventeur: NEFTEL, Frédéric, CH-1000 Lausanne 9 (CH); BOUVIER, Bernard, F-95610 Eragny (FR)
(74) Mandataire: Dronne, Guy
(86) Numéro de dépôt international: FR9700771
(87) Numéro de publication internationale: WO97041918

(56) Documents cités:
- EP-A- 0 405 097
- US-A- 4 248 401
- US-A- 5 290 239
- US-A- 5 437 635

## Description

La présente invention a pour objet un dispositif d'obturation par pincement d'un tube souple.

De façon plus précise, l'invention concerne un dispositif mécanique qui permet d'obturer totalement par pincement, afin d'interrompre la circulation d'un liquide, un tube ou une tubulure souple, notamment du type utilisé dans le domaine médical pour administrer à des patients des médicaments ou autres agents similaires par voie de perfusion.

On connaît déjà dans le domaine médical des dispositifs qui permettent d'obturer une telle tubulure souple par déplacement rectiligne d'une pièce qui comporte une fente rectiligne se terminant à une de ses extrémités par un évidement circulaire. Lorsque la tubulure est disposée dans l'évidement circulaire, on a un écoulement du liquide normal, en revanche, lorsqu'on déplace en translation linéaire la pièce, la fente réalise un pincement de la tubulure interrompant ainsi la circulation du liquide. Toutefois, avec de tels systèmes, l'obturation de la tubulure souple est peu fiable car elle risque de s'accompagner de déformation ou de formation de plis dans la paroi de la tubulure qui maintient ainsi à l'intérieur de cette tubulure une circulation résiduelle inadmissible. En outre, un tel système est relativement encombrant.

Un tel dispositif d'obturation par pincement d'un tube souple est utilisable dans le domaine médical, en particulier en liaison avec une pompe péristaltique permettant la mise en circulation sous pression d'un liquide entre une poche de stockage du liquide et une ligne de perfusion. Ces pompes péristaltiques sont de faible dimension et il est donc intéressant que le dispositif d'obturation soit lui-même de faible dimension et adapté à la structure d'une telle pompe péristaltique.

Les dispositifs d'obturation de l'art antérieur, notamment décrits dans les brevets américains 5,437,635 et 5,290,239, non seulement ne permettent pas une obturation optimale de la circulation du liquide dans le tube mais en outre, ils ne sont pas adaptés à une utilisation en liaison avec une pompe péristaltique ambulatoire de faible dimension.

Les pompes péristaltiques sont bien connues dans ce domaine.

De telles pompes péristaltiques sont notamment décrites dans la demande de brevet WO-95/31643 publiée le 23 Novembre 1995 au nom du déposant. De tels dispositifs de pompes sont constitués essentiellement d'un module pompe proprement dit ou cassette qui comporte des galets de pression coopérant avec un élément de tube déformable qui peut être inséré de façon amovible dans un bloc moteur, ce bloc moteur permettant l'entraînement en rotation des galets de compression et donc le fonctionnement de la pompe péristaltique. Dans ce cas, on comprend que, lorsque le moteur est arrêté et que l'on extrait la partie pompe proprement dite du carter du moteur, les galets de la pompe n'assurent plus leur fonction d'étanchéité locale sur l'élément de tube et une circulation du liquide à travers la pompe et donc dans les tubes associés peut se produire par gravité. Il est donc, dans ce cas particulier, strictement nécessaire de prévoir, associé à cette pompe péristaltique, un dispositif mécanique d'obturation du tube pour éviter tout accident lié à une mauvaise manipulation de la cassette par le personnel hospitalier ou le malade.

Un objet de la présente invention est de fournir un dispositif d'obturation par pincement d'un tube souple, notamment mais non exclusivement associé à une pompe péristaltique qui permette une interruption effective et totale du débit, tout en étant de préférence compatible avec la pompe péristaltique.

Pour atteindre ce but, selon l'invention, le dispositif d'obturation par pincement d'un tube souple, la zone du tube à obturer présentant un axe XX', se caractérise en ce qu'il comprend:
une pièce mobile d'obturation présentant une fente, ladite fente présentant une première extrémité et une deuxième extrémité, ladite deuxième extrémité étant raccordée à un évidement dont les dimensions sont au moins égales à celles de la section droite du tube, ladite fente ayant à proximité de sa première extrémité une largeur apte à réaliser le pincement complet dudit tube, la deuxième extrémité de la fente ayant une largeur qui va en augmentant pour se raccorder audit évidement, ledit dispositif se caractérisant en ce que ladite fente est en forme d'arc de cercle de rayon R, et en ce qu'il comprend en outre :
des moyens pour définir un axe de rotation (YY') fixe par rapport au tube souple à obturer et sensiblement parallèle audit axe XX' du tube;
des moyens pour guider en rotation ladite pièce d'obturation autour dudit axe de rotation de telle manière que ladite fente se déplace globalement sur un cercle dont le rayon est égal au rayon R, centré sur ledit axe de rotation et disposé dans un plan orthogonal audit axe de rotation, lesdits moyens comprenant un bras dont une première extrémité est montée pivotante autour dudit axe de rotation et dont la deuxième extrémité est solidaire de ladite pièce d'obturation; et
des moyens pour déplacer en rotation ladite pièce d'obturation entre une première position où le tube traverse librement ledit évidement de la pièce d'obturation et une deuxième position dans laquelle ledit tube est pincé dans la première extrémité de ladite fente.

On comprend que, grâce au déplacement circulaire de la pièce d'obturation qui comporte la fente d'obturation elle-même circulaire, on obtient une obturation effective et complète du tube grâce au déplacement de cette fente d'obturation selon un mouvement circulaire.

De préférence, le dispositif comprend en outre des moyens fixes pour sensiblement immobiliser ledit tube selon la direction de déplacement de ladite pièce d'obturation. De préférence encore, le dispositif comprend des moyens pour verrouiller ladite pièce d'obturation dans sa deuxième position.

Selon un mode préféré de mise en oeuvre, le dispositif pour l'obturation du tube de sortie d'une pompe péristaltique qui comprend un boîtier, une première buse cylindrique de raccordement à un tube d'entrée et une deuxième buse de raccordement audit tube de sortie, lesdites buses ayant des axes sensiblement parallèles, se caractérise en ce que ledit dispositif comprend un premier ensemble monté fixe sur ladite deuxième buse pour définir lesdits moyens de maintien, lesdits moyens de verrouillage et une partie desdits moyens de guidage en rotation et un deuxième ensemble monté pivotant autour de ladite première buse pour définir ledit bras, ladite pièce d'obturation, une partie des moyens de guidage en rotation et les moyens pour déplacer en rotation ladite pièce d'obturation.

On comprend que, dans cette application particulière, le mode de réalisation du dispositif d'obturation est particulièrement bien adapté à un emploi avec une pompe péristaltique puisque le dispositif se réduit à deux pièces très simples utilisant une des buses de la pompe péristaltique comme organe de fixation d'une des parties du dispositif d'obturation et l'autre buse de la pompe péristaltique comme axe de pivotement de la deuxième partie mobile du dispositif d'obturation.

D'autres caractéristiques et avantages de l'invention apparaîtront mieux à la lecture de la description qui suit d'un mode préféré de réalisation de l'invention donné à titre d'exemple non limitatif. La description se réfère aux figures annexées sur lesquelles:
- la figure 1 est une vue simplifiée montrant le montage des deux parties du dispositif d'obturation sur le boîtier d'une pompe péristaltique;
- la figure 2 est une vue de droite en perspective d'un premier mode de réalisation du dispositif d'obturation dans la position de fonctionnement normal;
- la figure 3 est une vue de gauche du dispositif d'obturation de la figure 2 en perspective, l'obturateur étant également en position ouverte;
- la figure 4 est une vue en perspective du dispositif d'obturation en position d'obturation;
- la figure 5 est une vue en perspective de la seule partie de maintien du dispositif d'obturation de la figure 2;
- la figure 6a est une vue de dessus de la partie de maintien du dispositif d'obturation de la figure 2;
- la figure 6b est une vue en coupe selon ligne BB de la figure 6a;
- les figures 7 et 8 sont des vues en perspective d'un deuxième mode de réalisation de la partie de maintien;
- la figure 9a est une vue de dessus du deuxième mode de réalisation de la partie de maintien;
- la figure 9b est une vue en coupe selon la ligne B-B de la figure 9a; et
- la figure 10 est un schéma montrant le principe de fonctionnement du dispositif d'obturation.

Dans la description qui suit, on va décrire un mode de réalisation du dispositif d'obturation destiné à être monté sur le boîtier d'une pompe péristaltique. Il va cependant de soi que l'invention pourrait prendre d'autres formes de réalisation adaptables à l'obturation d'une tubulure souple, notamment utilisée dans le domaine médical pour interrompre de façon mécanique la circulation du liquide dans cette tubulure.

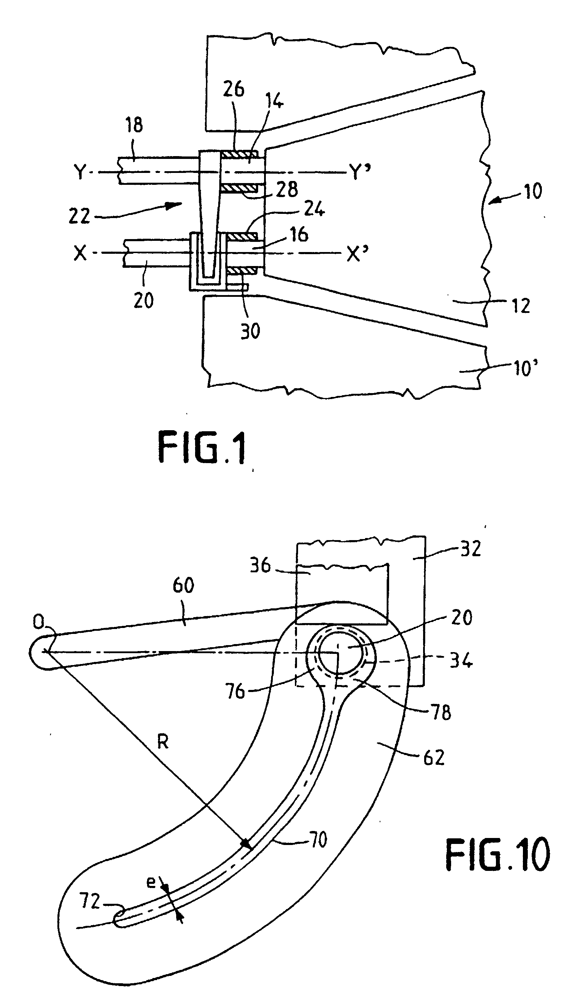

La figure 1 montre de façon simplifiée le montage des deux parties du dispositif d'obturation sur une pompe péristaltique 10. Sur cette figure, on a représenté schématiquement le boîtier 12 de la pompe péristaltique avec ses deux buses respectivement d'entrée 14 et de sortie 16 sur lesquelles on vient fixer la tubulure d'entrée 18 et la tubulure de sortie 20. On a également représenté schématiquement le carter 10' du moteur de la pompe dans lequel la pompe ou cassette peut être engagée de façon amovible. Dans la description qui suit, c'est donc la tubulure de sortie 20 qui doit être obturée mécaniquement. Les buses 14 et 16 ont chacune une forme cylindrique et présentent des axes XX' et YY' qui sont parallèles entre eux.

Comme on l'a déjà indiqué, le dispositif d'obturation qui porte la référence générale 22 est constitué de deux parties, d'une part, une partie fixe 24 servant essentiellement au maintien du tube 20 à obturer et au guidage de la deuxième partie 26 du dispositif d'obturation qui, elle, consiste essentiellement en une pièce pivotante d'obturation du tube.

Dans le mode de montage préféré, la partie 26 comportant la pièce d'obturation est montée pivotante autour de l'axe YY', par l'intermédiaire d'un manchon cylindrique 28, alors que la pièce 24 est montée fixe sur la buse 16 également par l'intermédiaire d'un manchon cylindrique 30. La figure 1 a simplement pour but d'exposer le montage du dispositif et ne représente donc pas en détail celui-ci.

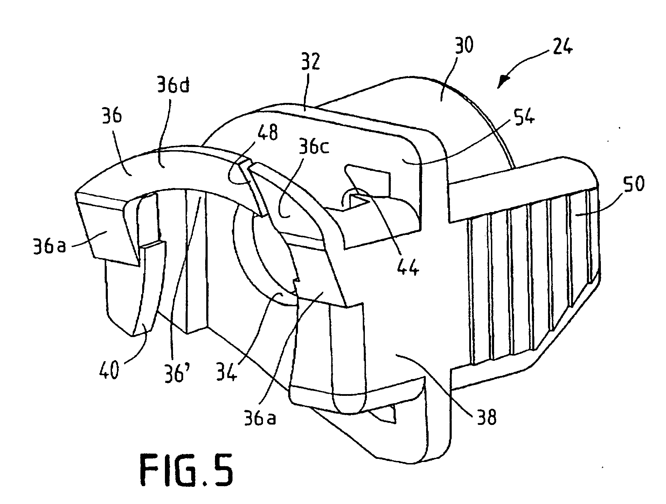

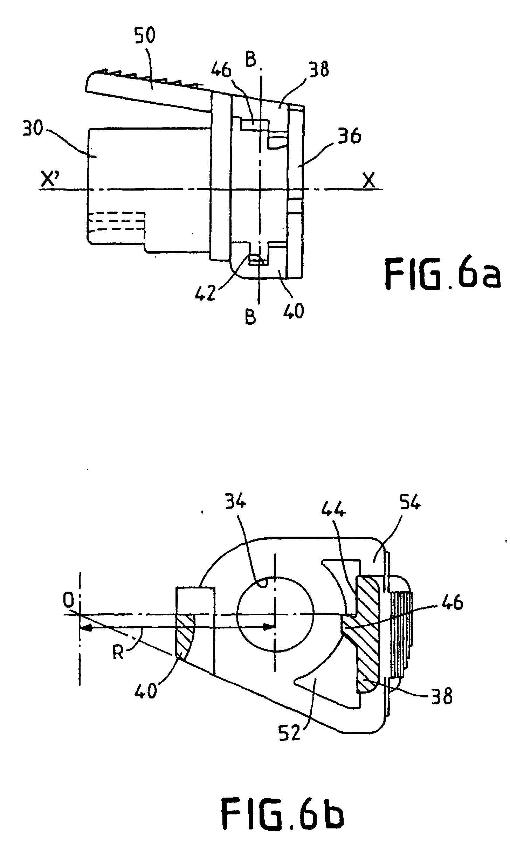

En se référant tout d'abord aux figures 5 et 6, on va décrire un premier mode préféré de réalisation de la partie de maintien 24.

Celle-ci est essentiellement constituée par une plaque 32 solidaire du manchon de fixation 30 et orthogonale à l'axe XX'. Cette plaque est munie d'un orifice circulaire 34 dont le diamètre est sensiblement égal au diamètre externe du tube à obturer. En avant de la plaque 32 par rapport au manchon 30, on trouve un organe de maintien du tube 36 qui a sensiblement la forme d'un arc de cercle. Cette pièce est raccordée à ses deux extrémités 36a et 36b aux deux extrémités de la plaque 32 par l'intermédiaire de portions de raccordement 38 et 40. Ainsi, il existe un espace libre entre la plaque 32, l'organe de maintien 36 et les portions de raccordement 38 et 40 qui, comme on l'expliquera ultérieurement, sert au passage de la pièce d'obturation. Comme le montrent les figures, les portions de raccordement 38 et 40 sont munies chacune dans leur face interne d'une rainure de guidage respectivement 42 et 44 qui ont de plus une forme d'arc de cercle centré sur l'axe YY' de l'autre buse de la pompe péristaltique. Comme on le voit mieux sur la figure 6b, la rainure 44 de la portion de raccordement 38 est munie d'un ergot 46 dont on expliquera ultérieurement la fonction. En outre, comme on le voit, la partie en arc de cercle 36 de l'organe de maintien est munie d'une fente inclinée 48 définissant ainsi deux parties 36c et 36d de l'organe de maintien 36.

Comme on le voit également sur les figures 5 et 6a, la portion de raccordement 38 est prolongée au-delà de la plaque 32 par une languette d'actionnement 50, cette languette 50 prolongeant exactement la portion de raccordement 38. On voit également que la plaque 32 est en fait munie d'un évidement 52 qui définit ainsi une portion verticale de la plaque 54 sur laquelle sont fixées de part et d'autre la portion de raccordement 38 et la languette 50. On comprend que, grâce à l'évidement 52, en appuyant sur la languette 50, on peut provoquer par déformation élastique un pivotement de la partie verticale 54 de la plaque, ce qui entraîne un pivotement correspondant de la portion de raccordement 38 pour des raisons qui seront explicitées ultérieurement. On comprend également que ce pivotement de la portion de raccordement 38 est rendu possible par la présence de la fente inclinée 48 ménagée dans l'organe de maintien 36.

Il faut également préciser que l'organe de maintien 36 est disposé de telle manière que sa face inférieure 36' soit sensiblement au contact du bord supérieur du tube à obturer.

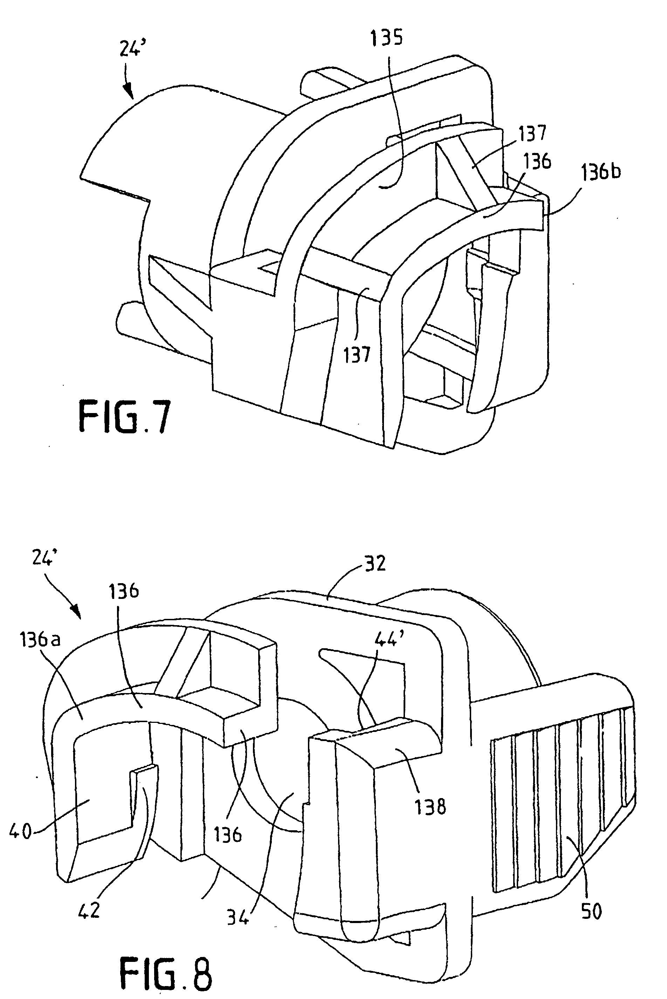

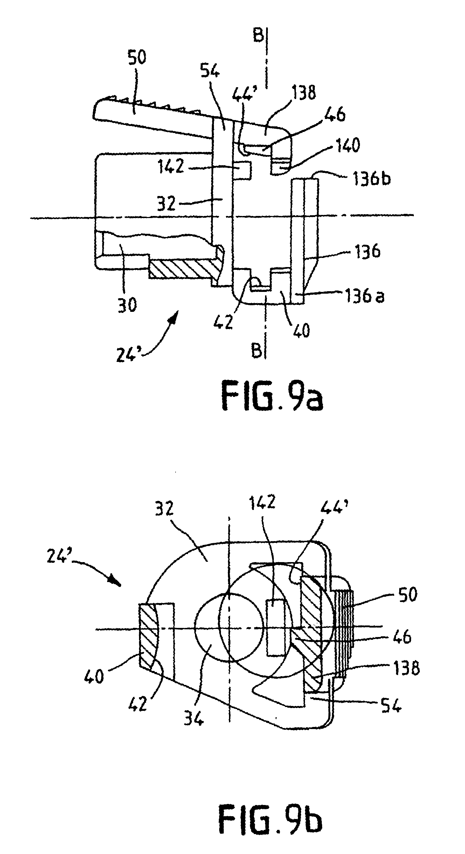

En se référant maintenant aux figures 7 à 9, on ve décrire un deuxième mode de réalisation de la partie de maitien qui porte la référence 24'.

Elle se distingue de la partie de maintien 24 en ce qui concerne la réalisation de l'organe de maintien 36 et celle de la nervure 44 ménagée dans la pièce de raccordement 38.

Le nouvel organe de maintien 136 est seulement raccordé par son extrémité 136a à la portion de raccordement 40. Son autre extrémité 136b est libre. L'organe de maintien 136 est donc monté en porte-à-faux. De préférence, il comporte des nervures de rigidification 135 et 137. Il remplit exactement la même fonction que l'organe 36 en empêchant le soulèvement du tube 20.

La gorge de guidage 44 de la figure 5 est remplacée par la gorge 44' qui est définie par le retour 140 de la pièce de raccordement 138 et par une nervure 142 faisant saillie dans la face avant de la plaque 32 en regard du retour 140. Dans cette nervure 44', on retrouve le verrou 46.

Dans ce mode de réalisation, on comprend que la pièce de raccordement 138 peut pivoter librement autour de la partie 54 de la plaque 32 sous l'effet de l'actionnement de la languette 50 puisque cette pièce de raccordement n'est pas reliée à l'organe de maintien 136.

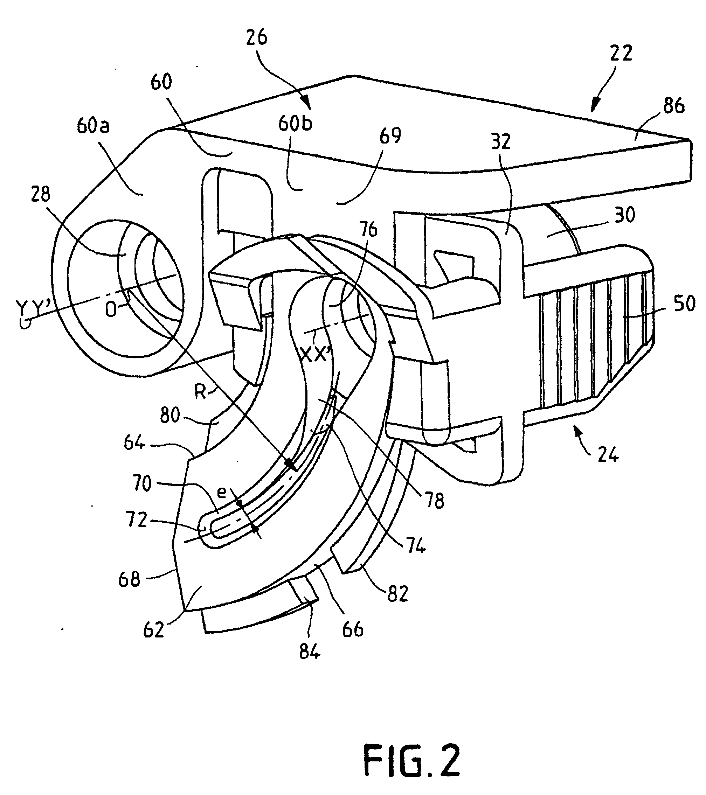

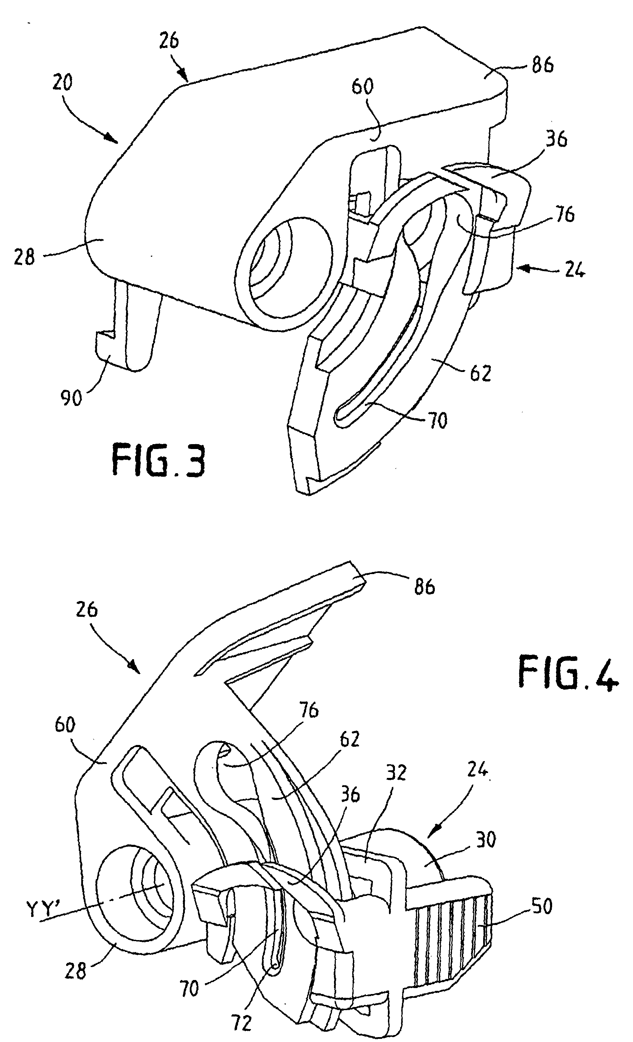

En se référant maintenant plus particulièrement aux figures 2 et 3, on va décrire la partie mobile d'obturation 26 du dispositif d'obturation qui peut être utilisée avec les deux modes de réalisation de la partie de maintien 24 ou 24'.

Cette partie 26 est essentiellement constituée par le manchon 28 qui est monté pivotant sur la buse 14. En d'autres termes, la partie d'obturation 26 peut donc pivoter autour de l'axe YY' qui est parallèle à l'axe XX' de la tubulure à obturer et, plus précisément, cette pièce peut pivoter autour de cet axe sensiblement dans un plan évidemment perpendiculaire à cet axe et donc perpendiculaire à l'axe du tube à obturer. La pièce 26 est essentiellement constituée par une portion 60 constituant un bras qui est, à sa première extrémité 60a, raccordée au manchon 28 et qui est, à sa deuxième extrémité 60b, raccordée à une pièce d'obturation 62. Cette pièce d'obturation 62 a la forme générale d'un arc de cercle limité par un bord circulaire interne 64 et par une bord circulaire externe 66, la pièce d'obturation 62 présentant une première extrémité libre 68 et une deuxième extrémité 69 raccordée à l'extrémité 60b du bras 60.

Les bords circulaires 64 et 66 de la pièce d'obturation 62 sont des arcs de cercle concentriques dont le centre commun est le point O disposé sur l'axe de pivotement YY'.

La plaque en forme d'arc de cercle constituant la pièce d'obturation 62 est munie dans sa partie médiane d'une fente 70. Cette fente présente une première extrémité 72 proche de l'extrémité 68 de la pièce 62 et une deuxième extrémité 74. La fente 70 est prolongée par un évidement 76 de forme circulaire de préférence dont le diamètre est légèrement supérieur au diamètre externe du tube à obturer. L'extrémité 74 de la fente 70 se raccorde tangentiellement dans la zone 78 à l'évidement circulaire 76. La fente 70, dans sa partie courante, a une largeur e qui est sensiblement égale ou de préférence légèrement inférieure au double de l'épaisseur de la paroi du tube à obturer. La ligne moyenne de la fente 70 et le centre de l'évidement 76 sont disposés sur un arc de cercle de rayon R et centré sur l'axe YY'. Les lèvres de la fente 70 ont de préférence, dans un plan de coupe perpendiculaire au plan de la pièce 62, une forme arrondie ou similaire ou pour le moins une forme dépourvue de point singulier afin d'éviter les risques de détérioration du tube.

La figure 2 montre également que les bords 64 et 66 de la pièce d'obturation 62 sont munis de nervures en forme d'arc de cercle respectivement référencées 80 et 82 qui coopèrent avec les gorges 44 ou 44' et 42 ménagées dans les portions de maintien de la partie de maintien 24. On comprend qu'ainsi, lors du pivotement de la pièce d'obturation 62 autour de son axe YY', cette pièce est en plus guidée par la coopération des nervures 80, 82 avec les gorges 42, 44 ou 44'. Elle a donc un déplacement qui se produit dans un plan rigoureusement perpendiculaire à l'axe XX' du tube.

On voit, sur la figure 2, que la nervure 82 comporte un creux formant discontinuité 84 qui a une longueur suffisante pour permettre l'engagement de l'ergot 46. Le creux 84 est disposé de telle manière que l'ergot 46 pénètre dans l'évidement 84 lorsque la pièce d'obturation 62 est dans sa position représentée sur la figure 4, c'est-à-dire dans sa position d'obturation, on obtient ainsi un verrouillage de la pièce d'obturation dans sa position d'obturation. En outre, on voit que la nervure 82 a une épaisseur qui augmente lorsqu'on se rapproche de l'extrémité 68 de la pièce 62. Cela permet d'obtenir effectivement le verrouillage grâce à la possibilité de pivotement de la portion de raccordement 38 ou 138.

L'utilisation du dispositif d'obturation qui vient d'être décrit se fait de la manière suivante:

Lors du fonctionnement normal de la pompe péristaltique, la partie d'obturation 26 est dans la position représentée sur les figures 2 et 3, c'est-à-dire que le tube de sortie 20 est libre et passe à travers l'orifice 34, l'évidement 76 de la pièce d'obturation et l'organe de maintien 36 ou 136. Lorsque l'on veut commander l'obturation du tube à l'aide du dispositif, on agit sur la plaquette de commande 86 solidaire du bras 60. En soulevant cette plaquette 86, on provoque le mouvement de pivotement vers le haut de l'organe d'obturation. Le tube étant maintenu vers le haut par la paroi de l'orifice 34 et par la pièce de maintien 36 ou 136, le soulèvement de la pièce d'obturation 62 provoque l'engagement de la partie inférieure du tube dans la partie 78 évasée de la fente 70. On obtient ainsi un début de pincement du bord inférieur du tube dans la zone convergente 78 puis ce pincement est complété au fur et à mesure que la pièce d'obturation est relevée jusqu'à ce que cette pièce arrive en position finale, position dans laquelle le tube est totalement pincé et ses parois légèrement écrasées de telle manière qu'on obtienne effectivement une obturation totale du tube.

Il est important de souligner que, grâce au déplacement circulaire de la fente d'obturation conjugué à la forme circulaire de cette fente et au maintien du tube par la pièce 24 ou 24', on obtient effectivement un écrasement total des parois du tube sans qu'il risque de subsister des replis qui autoriseraient encore une circulation réduite du liquide. La figure 10 illustre ce principe de fonctionnement.

En outre, lorsque la pièce d'obturation a été amenée dans sa position finale, l'ergot de verrouillage 46 pénètre dans le creux 84 de la pièce d'obturation assurant ainsi le maintien de l'obturation du tube. Pour libérer la pièce 62, il faut agir sur la languette 50 qui provoque l'écartement de l'ergot 46 et donc la possibilité d'abaisser à nouveau la pièce 62.

De préférence, comme le montre la figure 3, la partie d'obturation 26 comporte sur son manchon 28 à sa partie inférieure, un crochet 90 faisant saillie hors du manchon. Lorsque le dispositif d'obturation est dans la position de fonctionnement normal, c'est-à-dire dans les positions représentées sur les figures 2 et 3, le crochet 90 coopère avec une partie conjuguée (non représentée) du carter 10' du moteur associée à la pompe péristaltique 10, il est donc impossible d'extraire la cassette de la pompe péristaltique 10 lorsque l'obturateur est dans la position ouverte. En revanche, lorsque la pièce d'obturation 62 a été amenée dans la position représentée sur la figure 4, c'est-à-dire la position d'obturation, position dans laquelle la pièce 62 est verrouillée sur la partie 24 du dispositif d'obturation, le crochet 90 est libéré et il est donc possible d'enlever librement la cassette 10 de la pompe péristaltique hors du carter du moteur.

## Revendications

1. Dispositif d'obturation par pincement d'un tube souple (20), la zone du tube à obturer présentant un axe XX' comprenant une pièce mobile d'obturation (62) présentant une fente (70), ladite fente présentant une première extrémité (72) et une deuxième extrémité (74), ladite deuxième extrémité étant raccordée à un évidement (76) dont les dimensions sont au moins égales à celles de la section droite du tube, ladite fente ayant à proximité de sa première extrémité une largeur apte à réaliser le pincement complet dudit tube, la deuxième extrémité de la fente ayant une largeur qui va en augmentant pour se raccorder audit évidement, ledit dispositif se caractérisant en ce que ladite fente (70) est en forme d'arc de cercle de rayon R, et en ce qu'il comprend en outre :
des moyens pour définir un axe de rotation (YY') fixe par rapport au tube souple à obturer et sensiblement parallèle audit axe XX' du tube;
des moyens (60, 42, 44, 44', 80, 82) pour guider en rotation ladite pièce d'obturation autour dudit axe de rotation de telle manière que ladite fente se déplace globalement sur un cercle dont le rayon est égal au rayon R, centré sur ledit axe de rotation et disposé dans un plan orthogonal audit axe de rotation, lesdits moyens comprenant un bras (60) dont une première extrémité (60a) est montée pivotante autour dudit axe de rotation et dont la deuxième extrémité (60b) est solidaire de ladite pièce d'obturation; et
des moyens (86) pour déplacer en rotation ladite pièce d'obturation (62) entre une première position où le tube traverse librement ledit évidement de la pièce d'obturation et une deuxième position dans laquelle ledit tube est pincé dans la première extrémité de ladite fente.

2. Dispositif selon la revendication 1, **caractérisé en ce qu'**il comprend en outre des moyens fixes (24, 24') pour sensiblement immobiliser ledit tube selon la direction de déplacement de ladite pièce d'obturation.

3. Dispositif selon la revendication 2, **caractérisé en ce qu'**il comprend en outre des moyens (50, 46, 84) pour verrouiller ladite pièce d'obturation dans sa deuxième position.

4. Dispositif selon l'une quelconque des revendications 1 à 3 pour l'obturation du tube de sortie (20) d'une pompe péristaltique comprenant un boîtier (10), une première buse cylindrique (14) de raccordement à un tube d'entrée et une deuxième buse (16) de raccordement audit tube de sortie, lesdites buses ayant des axes sensiblement parallèles, **caractérisé en ce que** ledit dispositif comprend un premier ensemble (24, 24') monté fixe sur ladite deuxième buse pour définir lesdits moyens de maintien, lesdits moyens de verrouillage et une partie desdits moyens de guidage en rotation et un deuxième ensemble (26) monté pivotant autour de ladite première buse pour définir ledit bras, ladite pièce d'obturation, une partie des moyens de guidage en rotation et les moyens pour déplacer en rotation ladite pièce d'obturation.

5. Dispositif selon la revendication 4, **caractérisé en ce que** ladite pièce d'obturation (62) est constituée par une plaque en forme d'arc de cercle limitée par un bord circulaire interne (64) et un bord circulaire externe (66) et présentant une première extrémité (68) correspondant à la première extrémité de la fente (70) et une deuxième extrémité (69) correspondant audit évidement (76), la deuxième extrémité (60b) dudit bras étant raccordée à une extrémité de ladite pièce d'obturation.

6. Dispositif selon la revendication 5, **caractérisé en ce que** lesdits moyens de maintien comprennent une première plaque (32) solidaire de ladite deuxième buse et munie d'un orifice (34) dont le diamètre est légèrement supérieur au diamètre externe dudit tube et disposé dans un plan orthogonal à l'axe dudit tube.

7. Dispositif selon la revendication 6, **caractérisé en ce que** les moyens de maintien comprennent en outre un organe de maintien (36) décalé axialement par rapport à ladite plaque et solidaire de ladite plaque de telle manière que ladite pièce d'obturation (62) puisse se déplacer entre ladite plaque et ledit organe de maintien, ledit organe de maintien étant sensiblement en contact avec ledit tube (20) et apte à immobiliser ledit tube lorsque ladite pièce d'obturation est déplacée de sa première à sa deuxième position.

8. Dispositif selon la revendication 7, **caractérisé en ce que** ledit organe de maintien (36) a la forme d'un arc de cercle dont une première extrémité est reliée à ladite plaque (32) par une première portion de raccordement (38) et dont la deuxième extrémité est raccordée à ladite plaque par une deuxième portion de raccordement (40).

9. Dispositif selon la revendication 8, **caractérisé en ce que** ledit organe de maintien (36) présente une fente (48) entre ses première et deuxième extrémités.

10. Dispositif selon la revendication 9, **caractérisé en ce que** lesdits moyens de maintien comprennent en outre un organe de maintien (136) décalé axialement par rapport à ladite plaque et solidaire de la plaque par une de ses extrémités, de telle manière que ladite pièce d'obturation (62) puisse se déplacer entre ladite plaque et ledit organe de maintien, ledit organe de maintien étant sensiblement en contact avec ledit tube (20) et apte à immobiliser ledit tube lorsque ladite pièce d'obturation est déplacée de sa première à sa deuxième position.

11. Dispositif selon la revendication 10, **caractérisé en ce que** ledit organe de maintien a la forme d'une portion d'arc de cercle dont une extrémité est libre et dont l'autre extrémité est reliée à ladite plaque par une première portion de raccordement et **en ce qu'**il comprend en outre une deuxième portion de raccordement solidaire de ladite plaque et faisant saillie hors de ladite plaque.

12. Dispositif selon l'une quelconque des revendication 8 et 11, **caractérisé en ce que** les bords interne et externe de la pièce d'obturation (62) sont munis d'une nervure (80, 82) apte à coopérer avec des moyens formant gorge (42, 44, 44') en forme d'arc de cercle ménagés dans chaque portion de raccordement des moyens de maintien.

13. Dispositif selon la revendication 11, **caractérisé en ce que** lesdits moyens de verrouillage comprennent un ergot (46) ménagé dans les moyens formant gorge (44, 44') de la deuxième portion de raccordement (38, 138) apte à coopérer avec un creux (84) ménagé dans la nervure (82) associée de ladite pièce d'obturation, ladite portion de raccordement (38, 138) étant prolongée par une languette de déverrouillage (50), la liaison (54) entre la portion de raccordement et la languette d'une part et un bord de la plaque d'autre part constituant un axe de pivotement de la partie de raccordement par rapport à la plaque, l'actionnement de ladite languette permettant le pivotement temporaire de la portion de raccordement pour permettre l'échappement dudit ergot hors dudit creux.

14. Dispositif selon l'une quelconque des revendications 3 à 13 pour une pompe péristaltique (10) dont le boîtier est engagé de façon amovible dans le carter (10') d'un moteur d'entraînement, **caractérisé en ce que** les premier (24, 24') et deuxième ensemble (26) sont chacun montés sur les buses (14, 16) par l'intermédiaire de manchons (28, 30), le manchon (28) du deuxième ensemble (26) étant monté pivotant sur la buse associée.

15. Dispositif selon la revendication 14, **caractérisé en ce que** ledit manchon monté pivotant présente un organe de verrouillage (90) apte à coopérer avec un organe conjugué du carter (10') lorsque la pièce d'obturation (62) n'est pas dans sa deuxième position.

## Patentansprüche

1. Vorrichtung zum Verschließen eines Schlauchs (20) durch Quetschen, wobei der zu verschließende Bereich des Schlauchs eine Achse XX' aufweist, die ein bewegliches Verschließteil (62) umfasst, das einen Spalt (70) aufweist, wobei der Spalt ein erstes Ende (72) und ein zweites Ende (74) aufweist, wobei das zweite Ende mit einer Erweiterung (76) in Verbindung steht, deren Abmessungen mindestens gleich denen des Querschnitts des Schlauchs sind, wobei der Spalt in der Nähe seines ersten Endes eine Breite hat, bei der das vollständige Zuquetschen des Schlauchs bewirkt werden kann, und wobei das zweite Ende des Spalts eine Breite hat, die sich langsam erhöht, um an die Erweiterung anzuschließen, wobei die Vorrichtung **dadurch gekennzeichnet ist, dass** der Spalt (70) die Form eines Kreisbogens mit dem Radius R hat, und dass sie ferner aufweist:
Mittel, um eine bezogen auf den zu verschließenden Schlauch feste Drehachse (YY') festzulegen, die im Wesentlichen parallel zur Achse XX' des Schlauchs verläuft;
Mittel (60, 42, 44, 44', 80, 82) zum Führen des Teils zum Verschließen um die Drehachse bei der Drehung derart, dass sich der Spalt im Ganzen in einem Kreis bewegt, dessen Radius gleich dem Radius R ist, und um die Drehachse zentriert ist und in einer zur Drehachse senkrechten Ebene angeordnet ist, wobei die Mittel einen Arm (60) aufweisen, von dem ein erstes Ende (60a) um die Drehachse schwenkbar befestigt ist, und von dem ein zweites Ende (60b) mit dem Verschließteil verbunden ist; und
Mittel (86) zum drehenden Bewegen des Teils (62) zum Verschließen zwischen einer ersten Stellung, bei der das Rohr die Erweiterung des Verschließstücks ungehindert durchquert, und einer zweiten Stellung, bei der der Schlauch im ersten Ende des Spalts gequetscht ist.

2. Vorrichtung nach Anspruch 1, **dadurch gekennzeichnet, dass** sie ferner feste Mittel (24, 24') umfasst, um den Schlauch entsprechend der Bewegungsrichtung des Verschließteils merklich festzuhalten.

3. Vorrichtung nach Anspruch 2, **dadurch gekennzeichnet, dass** sie ferner Mittel (50, 46, 84) zum Verriegeln des Verschließteils in seiner zweiten Stellung umfasst.

4. Vorrichtung nach einem der Ansprüche 1 bis 3 zum Verschließen des Ausgangsrohrs (20) einer Quetschschlauchpumpe, welche ein Gehäuse (10), eine erste zylindrische Düse (14) zum Verbinden mit einem Eingangsrohr und eine zweite Düse (16) zum Verbinden mit dem Ausgangsrohr umfasst, wobei die Düsen im Wesentlichen parallel zueinander verlaufende Achsen aufweisen, **dadurch gekennzeichnet, dass** die Vorrichtung eine erste, fest an der zweiten Düse befestigte Einheit (24, 24') zum Festlegen der Haltemittel, die Verriegelungsmittel und einen Teil der Mittel zum Führen bei der Drehung und eine zweite, um die erste Düse schwenkbar befestigte Einheit (26) zum Festlegen des Arms, das Verschließteil und einen Teil der Mittel zum Führen bei der Drehung und die Mittel zum drehenden Verschieben des Verschließteils umfasst.

5. Vorrichtung nach Anspruch 4, **dadurch gekennzeichnet, dass** das Verschließteil (62) aus einer Platte in Form eines Kreisbogens gebildet ist, der von einem inneren kreisförmigen Rand (64) und einem äußeren kreisförmigen Rand (66) begrenzt ist, und ein erstes Ende (68), das dem ersten Ende des Spalts (70) entspricht, und ein zweites Ende (69), das der Erweiterung (76) entspricht, umfasst, wobei das zweite Ende (60b) des Arms mit einem Ende des Verschließteils verbunden ist.

6. Vorrichtung nach Anspruch 5, **dadurch gekennzeichnet, dass** die Haltemittel eine erste Platte (32) umfassen, die mit der zweiten Düse verbunden ist und mit einer Öffnung (34) versehen ist, deren Durchmesser etwas größer als der äußere Durchmesser des Schlauchs ist, und die in einer zur Achse des Schlauchs senkrechten Ebene angeordnet ist.

7. Vorrichtung nach Anspruch 6, **dadurch gekennzeichnet, dass** die Mittel zum Halten ferner ein Halteteil (36) umfassen, das bezogen auf die Platte axial versetzt ist und mit der Platte derart verbunden ist, dass sich das Verschließteil (62) zwischen der Platte und dem Halteteil bewegen kann, wobei das Halteteil mit dem Schlauch (20) im Wesentlichen in Berührung steht und den Schlauch festhalten kann, wenn das Verschließteil von seiner ersten in seine zweite Stellung bewegt wird.

8. Vorrichtung nach Anspruch 7, **dadurch gekennzeichnet, dass** das Halteteil (36) die Form eines Kreisbogens hat, dessen eines Ende mit einem ersten Verbindungsabschnitt (38) mit der Platte (32) verbunden ist, und dessen zweites Ende über einen zweiten Verbindungsabschnitt (40) mit der Platte verbunden ist.

9. Vorrichtung nach Anspruch 8, **dadurch gekennzeichnet, dass** das Halteteil (36) zwischen seinem ersten und zweiten Ende einen Spalt (48) aufweist.

10. Vorrichtung nach Anspruch 9, **dadurch gekennzeichnet, dass** die Haltemittel ferner ein Halteteil (136) umfassen, das bezogen auf die Platte axial versetzt ist und über eines seiner Enden mit der Platte verbunden ist, derart, dass sich das Verschließteil (62) zwischen der Platte und dem Halteteil bewegen kann, wobei das Halteteil mit dem Schlauch (20) im Wesentlichen in Berührung steht und den Schlauch festhalten kann, wenn das Verschließteil aus seiner ersten Stellung in seine zweite Stellung bewegt wird.

11. Vorrichtung nach Anspruch 10, **dadurch gekennzeichnet, dass** das Halteteil die Form eines Teilkreisbogens hat, dessen eines Ende frei ist, und dessen anderes Ende über einen ersten Verbindungsabschnitt mit der Platte verbunden ist, und dass es ferner einen zweiten Verbindungsabschnitt umfasst, der mit der Platte in Verbindung steht und aus der Platte herausragt.

12. Vorrichtung nach einem der Ansprüche 8 oder 11, **dadurch gekennzeichnet, dass** der Innen- und Außenrand des Verschließteils (62) mit Rippen (80, 82) versehen sind, die mit eine Nut (42, 44, 44') in Form eines Kreisbogens bildenden Mitteln zusammenwirken können, welche in jedem Verbindungsabschnitt der Haltemittel vorgesehen sind.

13. Vorrichtung nach Anspruch 11, **dadurch gekennzeichnet, dass** die Verriegelungsmittel einen Vorsprung (46) umfassen, der in den eine Nut (44, 44') des zweiten Verbindungsabschnitts (38, 138) vorgesehen ist, die mit einer Vertiefung (84), die in der zu diesem Verschließteil zugehörigen Rippe (82) angeordnet ist, zusammenwirken kann, wobei der Verbindungsabschnitt (38, 138) durch eine Entriegelungszunge (50) verlängert ist, und wobei die Verbindung (54) zwischen einerseits dem Verbindungsabschnitt und der Zunge und andererseits einem Rand der Platte eine Schwenkachse des Verbindungsabschnitts bezüglich der Platte bilden, wobei die Betätigung der Zunge das zeitweilige Verschwenken des Verbindungsabschnitts ermöglicht, um das Austreten des Vorsprungs aus der Vertiefung zu ermöglichen.

14. Vorrichtung nach einem der Ansprüche 3 bis 13 für eine Quetschschlauchpumpe (10), deren Gehäuse in abnehmbarer Weise in das Gehäuse (10') eines Mitführmotors eingreift, **dadurch gekennzeichnet, dass** die erste (24, 24') und zweite Einheit (26) jeweils über Hülsen (28, 30) an den Düsen (14, 16) befestigt sind, wobei die Hülse (28) der zweiten Einheit (26) schwenkbar an der zugehörigen Düse befestigt ist.

15. Vorrichtung nach Anspruch 14, **dadurch gekennzeichnet, dass** die schwenkbar befestigte Hülse ein Verriegelungsteil (90) aufweist, das mit einem gekoppelten Teil des Gehäuses (10') zusammenwirken kann, wenn das Verschließteil (62) nicht in seiner zweiten Stellung ist.

## Claims

1. A closure device for closing a hose (20) by pinching, the zone of the hose to be closed having an axis XX' comprising a moving closure piece (62) having a slot (70), said slot having a first end (72) and a second end (74), said second end being connected to a recess (76) of dimensions that are not less than the dimensions of the right section of the hose, said slot having in the vicinity of its first end a width that is suitable for pinching said hose completely, the second end of the slot having a width that flares to connect with said recess, said device being **characterized in that** said slot (70) is in the form of a circular arc of radius R, and **in that** said device further comprises:
means for defining a fixed axis of rotation (YY') with respect to the hose to close that is substantially parallel to said axis (XX') of the hose;
means (60, 42, 44, 44', 80, 82) for guiding said closure piece in rotation about said axis of rotation in such a manner that said slot moves generally along a circle of radius equal to the radius R, centred on said axis of rotation, and disposed in a plane orthogonal to said axis of rotation, said means comprising an arm (60) having a first end (60a) pivotally mounted about said axis of rotation and having a second end (60b) secured to said closure piece; and
means (86) for rotating said closure piece (62) between a first position in which the hose passes freely through said recess of the closure piece and a second position in which said hose is pinched in the first end of said slot.

2. A device according to claim 1, **characterised in that** it further comprises fixed means (24, 24') for substantially preventing said hose from moving along the displacement direction of said closure piece.

3. A device according to claim 2, **characterised in that** it further includes means (50, 46, 84) for locking said closure piece in its second position.

4. A device according to any one of claims 1 to 3 for closing the outlet hose (20) of a peristaltic pump comprising a housing (10), a first cylindrical nozzle (14) for connection to an inlet hose and a second nozzle (16) for connection to said outlet hose, said nozzles having axes that are substantially parallel, the device being **characterised in that** it comprises a first assembly (24, 24') mounted in fixed manner on said second nozzle to define said holding means, said locking means, and a portion of said means for providing guidance in rotation, and a second assembly (26) pivotally mounted about said first nozzle to define said arm, said closure piece, a portion of the means for providing guidance in rotation, and the means for displacing said closure piece in rotation.

5. A device according to claim 4, **characterised in that** said closure piece (62) is constituted by a plate in the form of a circular arc defined by an inner circular edge (64) and an outer circular edge (66) and having a first end (68) corresponding to the first end of the slot (70) and a second end (69) corresponding to said recess (76), the second end (60b) of said arm being connected to one end of said closure piece.

6. A device according to claim 5, **characterised in that** said holding means comprise a first plate (32) secured to said second nozzle and provided with an orifice (34) of diameter slightly greater than the outside diameter of said tube and disposed in a plane orthogonal to the axis of said tube.

7. A device according to claim 6, **characterised in that** the holding means further comprise a holding member (36) offset axially relative to said plate and secured to said plate in such a manner that said closure piece (62) can move between said plate and said holding member, said holding member being substantially in contact with said hose (20) and suitable for preventing said hose from moving while said closure piece is being moved from its first position to its second position.

8. A device according to claim 7, **characterised in that** said holding member (36) is in the form of an arc of a circle having a first end connected to said plate (32) by a first connection portion (38) and having a second end connected to said plate by a second connection portion (40).

9. A device according to claim 8, **characterised in that** said holding member (36) has a slot (48) between its first and second ends.

10. A device according to claim 9, **characterised in that** said holding means further comprise a holding member (136) axially offset from said plate and secured to the plate via one of its ends in such a manner that said closure piece (62) is capable of moving between said plate and said holding member, said holding member being substantially in contact with said hose (20) and being suitable for preventing said hose from moving while said closure piece is being moved from its first position to its second position.

11. A device according to claim 10, **characterised in that** said holding member is in the form of a circularly arcuate portion having one end that is free and having its other end connected to said plate via a first connection portion, and **in that** it further comprises a second connection portion secured to said plate and projecting from said plate.

12. A device according to claim 8 or 11, **characterised in that** the inner and outer edges of the closure piece (62) are provided with respective ribs (80, 82) suitable for co-operating with groove-forming means (42, 44, 44') of circularly arcuate shape formed in each of the connection portions of the holding means.

13. A device according to claim 11, **characterised in that** said locking means comprise a stud (46) formed in the groove-forming means (44, 44') of the second connection portion (38, 138) and suitable for co-operating with a notch (84) formed in the associated rib (82) of said closure piece, said connection portion (38, 138) being extended by an unlocking tongue (50), the link (54) between the connection portion and firstly the tongue and secondly an edge of the plate constituting a pivot axis enabling the connection portion to pivot relative to the plate, action on said tongue enabling the connection portion to pivot in temporary manner so as to allow said stud to escape from said notch.

14. A device according to any one of claims 3 to 13, for a peristaltic pump (10) whose housing is engaged in removable manner in the case (10') of a drive motor, the device being **characterised in that** each of the first and second assemblies (24, 24'; 26) is mounted on a respective one of said nozzles (14, 16) by means of respective sleeves (28, 30), the sleeve (28) of the second assembly (26) being mounted to pivot on the associated nozzle.

15. A device according to claim 14, **characterised in that** said pivotally mounted sleeve has a locking member (90) suitable for co-operating with a complementary member of the case (10') when the closure piece (62) is not in its second position.
